# EUROPEAN PATENT SPECIFICATION

(11) **EP 1 174 152 B1**
(45) Date of publication and mention of the grant of the patent: **20.12.2006**
(21) Application number: 00302674.7
(22) Date of filing: 30.03.2000
(51) Int. Cl.: A61K 47/48

(54) **Inclusion complex of an opioid peptide with cyclodextrin**
Inklusionskomplex von Opioid-Peptid mit Cyclodextrin
Complexe d'inclusion d'un peptide opioide avec une cyclodextrin

(43) Date of publication of application: 23.01.2002
(73) Proprietor: COUNCIL OF SCIENTIFIC AND INDUSTRIAL RESEARCH, New Delhi 110 001 (IN)
(72) Inventor: Dwivedi, Anil Kumar, Central Drug Res. Inst., Lucknow 226001, U.P. (IN); Srivastava, Sudhir, Central Drug Res. Inst., Lucknow 226001, U.P. (IN); Srivastava, Jagdishwar S., Central Drug Res. Inst., Lucknow 226001, U.P. (IN); Haq, Wahajul, Central Drug Res. Inst., Lucknow 226001, U.P. (IN); Murhty, Puwada Sri R., Central Drug Res. Inst., Lucknow 226001, U.P. (IN); Singh, Satyawan, Central Drug Res. Inst., Lucknow 226001, U.P. (IN); Raghubir, Ram, Central Drug Res. Inst., Lucknow 226001, U.P. (IN); Asthana, Onkar Prasad, Central Drug Res. Inst., Lucknow 226001, U.P. (IN); Khanna, Madhu, Central Drug Res. Inst., Lucknow 226001, U.P. (IN)
(74) Representative: Wain, Christopher Paul

(56) References cited:
- US-A- 4 670 419
- DATABASE CHEMABS [Online] CHEMICAL ABSTRACTS SERVICE, COLUMBUS, OHIO, US; RAGHUBIR, R. ET AL: "Pharmacological profile of some [D-Ala2, MePhe4, Met5]-enkephalin- alkylamides, new potent analogs of met-enkephalin" retrieved from STN Database accession no. 97:175263 XP002148072 & ADV. BIOSCI. (1982), 38(CURR. STATUS CENT. ACTING PEPT.), 61-9,
- ZEZHI SAHAO ET AL.: "Cyclodextrins as Nasal Absorption Promoters of Insulin: Mechanistic Evaluations" PHARMACEUTICAL RESEARCH, vol. 9, no. 9, 1992, pages 1157-1163, XP000946411
- DATABASE EPODOC [Online] EUROPEAN PATENT OFFICE, THE HAGUE, NL; IN173568, MATHUR KRISHNA BEHARI;: " A process for the synthesis of n-substituted amides of l-tyrosyl-d-a-alanyl-glycyl-l-n-methylphen ylanyl-glycine " XP002149862

## Description

The present invention relates to inclusion complexes including the known opioid peptide, L-Tyrosyl-D-alanyl-glycyl-N-methylphenylalanyl-glycyl-isopropylamide.

Known narcotic analygesics, such as morphine, can be toxic, are addictive and patient tolerance can be relatively low. We have discovered peptide L-Tyrosyl-D-alanyl-glycyl-N-methylphenylalanyl-glycyl-isopropylamide which is a more potent analgesic, is less toxic, has a longer duration of action, greater efficacy, for which patients are more tolerant and which induces no or very limited dependency when compared with the narcotic analgesics. However L-Tyrosyl-D-alanyl-glycyl-N-methylphenylalanyl-glycyl-isopropylamide is poorly absorbed via the preferred oral route of administration, and this has limited its usefulness and more wide adoption as an analgesic.

Soon after the discovery of enkephalin by Huges et. Al [Huges et. Al, Nature 258, 577 (1975)], an endogenously occurring pentapeptide with morphinomimetic activity, structure activity relationship studies were undertaken world-wide with the objective of getting a synthetic congener that would be clinically acceptable pain killer as substitute to morphine. Since the analgesia evoked by enkephalins was only weak and transient following their administration by intra-cerebral route, greater emphasis was laid on the structural modification of the penta peptide which would lead to the peptide(s) capable of eliciting analgesis even after their systemic administration.

The two best enkephalin analogues that had undergone fairly extensive clinical studies so far, are the Sandoz compound FK -33-824 [Tyr-D-Ala-Gly-Met(o)-01 and the Lilly compound met-keohamid (Tyr-D-Ala-Gly-Phe-MeMet-NH2. [Von Graffenreid, B., del Pozo, E., Roubicek, J., Krebs, E., Poldinger W., Burmeister, P. and Kerp, L., Nature, 272, 729 (1978) and Frederickson, R.C.A., Smithwick, E. L., Shuman, R. and Bemis, K.G., Science, 211, 603, (1981) Frederickson, R.C.A., In 'Opioid Peptides: Molecular Pharmacology, Bio synthesis and analysis" Rapaka, R.S. and Hawks, R. L. eds. NIDA Research Monograph, *70 367. (1986) FK-33-824* gave only slight preference for µ-receptors and met-kephamid was found essentially non-elective for µ and δ receptors. A strong analgesic effect was exerted by both the compounds by systemic route of administration. However, due to a number of serious side effects produced by FK -33-824 it was not pursued for further developments as candidate analgesic drug. Relatively fewer side effects were observed with met-kephamid, but due to its hypotensive effect this compound was also finally abandoned.

Other enkephalin analogs Tyr-D-Met-Gly-Phe-Pro-NH₂ [Flodes, J., torok. K. Szekeley, J I. Bonenderg, J, Karezag, I , Tolna, J , Marosfi, S . Varadi, A , Gara, A, Ronai, A Z. and Szilaggi, G Life Sciences 33. Supp I, 769 (1983)] and Tyr-Arg-Gly-Phe(*p*-NO₂)-Pro-NH₂ (BW-143C) [*Fallenfant. R.L*., *Hardy, G.W., Lowe, L.A. Schneider, C. and Smith, T.W. Br. J Pharmacol, 93, 85, (1958) and Kriss, M.G., Gralla, R.J., Clark, R.A., Tysan, L.B. and Grosheu, S., J. Clin, Onclot., 6, 663, (1988)*] were also shown to be more potent analgesics than morphine but due to number of side effects these compounds were also dropped after initial clinical trials. Similarly, structure activity relationship studies were undertaken in our laboratory and an enkephalin analog Tyr-D-Ah-Gly-MePhe-Gly-NHC₃H₇ [Raghubir, R., Parnaik. G.K., Sharma, S.D, Mathur, K.B and Dhawan B.N., in recent progress in chemistry and biology of centrally acting peptides. Dhawan B.N, and Rapaka R.S. eds, 167, (1988) and Indian Patent no. 173568 19.10.1989] synthesised earlier in our laboratory was found to be more potent than morphine following systemic administration. This is a highly µ-receptor selective in central and peripheral assay and produces highly profound and long lasting analgesia (*C*. *Nath. G.K. Patnaik, W. Haq, K.B*. *Mathur, R.C. Srimal. B.N. Dhawan and F. Porteca Pharmacological Research*., *31*, *269-273*, *1995*) The compound and its process for the synthesis was first disclosed in Indian patent no. 173568 of 19.10.1989. Subsequently chronic and subacute toxicity studies on this compound were carried out and it is now disclosed that this compound is safe and did not produce any noticeable toxic side effects. This compound was also studied for their addiction liabilities and tolerance and found to elicit significantly reduced tolerance and addiction properties as compared to morphine. The compound is virtually devoid of any major CNS effects like sedation and respiratory depression; it is also virtually devoid of any significant cardiovascular effects. Therefore, this compound has a potential as centrally active analgesic agent, which can be used as a substitute to narcotic analgesics (Morphine and related substances). However, this compound upon oral administration produced poor response and an extremely high dose is required to obtain similar magnitude of response as observed after parenteral administration owing to its decomposition and poor absorption. The oral efficacy of the therapeutic agents is considered to be highly desirable, therefore, inspite of a profound analgesia and favourable pharmacological effect and almost devoid of toxic effects, the development of the peptide as drug is restricted due to its poor oral efficacy.

There has been tremendous emphasis on the development of innovative strategies for the oral delivery of peptide based drugs, (A Fasano. (1998) TIBTECH., 16, 152-157) with increased water solubility, dissolution, bioavailability and improved oral efficacy (Z. Shao, 1992). Cyclodextrins are reported in the literature that they increase water solubility, dissolution, bioavailability and stability of compound by forming inclusion complexes. [R. Krishnamoorthy and A.K. Mitra, Pharma. Res., 9: 1157-1163 (1992)]. Recently it was reported in the literature that the β-cyclodextrin inclusion complex increases the half life to Leu-enkephalin from 45 min to 75 min due to enzyme hydrolysis with leucine amino peptidase (W. J. Erwin., A.K. Dwivedi, P.A. Holbrook, and M. J. Dey, Pharma Res., 11, 1994, 1698-1703). Therefore, the preparation of inclusion complex of peptides and other substances with cyclodextrin is reported in the literature. The advantage of binding substances into inclusion complexes with cyclodextrin is also known in other substances. US Patent No. 4603123, 5840714 and 5855916 disclosed the increased therapeutic efficacy and reduced toxic effects of piroxicam, ibuproxam and acid base type drugs respectively. US 4,670,419 discloses a pharmaceutical composition containing a hydrophilic drug which is poorly absorbable through the gastrointestinal tract and cyclodextrin.

We have now discovered a way of formulating L-Tyrosyl-D-alanyl-glycyl-N-methylphenylalanyl-glycyl-isopropylamide whereby it is suitable for oral delivery in that it has increased water solubility, larger duration of action, improved analgesic activity, increased dissolution bioavailability and improved oral/transdermal efficacy over L-Tyrosyl-D-alanyl-glycyl-N-methylphenylalanyt-gycyl-isopropylamide alone.

According to one aspect, the invention provides an inclusion complex comprising a complex of L-Tyrosyl-D-alanyl-glycyl-N-methylphenylalanyl-glycyl-isopropylamide with a cyclodextrin, wherein the molar ratio between L-Tyrosyl-D-alanyl-glycyl-N-methylphenylalanyl-glcylpisopropylamide and the cyclodextrin is from 1:5 to 2:1. Preferably the cyclodextrin is selected from the group consisting of beta cyclodextrin, hydroxypropyl-beta cyclodextrin, dimethyl-beta cyclodextrin and hydroxyethyl-beta-cyclodextrin.

In one embodiment, the inclusion complex comprises L-Tyrosyl-D-alanylglycyl-N-methylphenylalanyl-glycyl-isopropylamide with beta-cyclodextrin.

In another embodiment, the inclusion complex comprises L-Tyrosyl-D-alanyl-glycyl-N-methylphenylalanyl-glycyl-isopropylamide with hydroxyethyl beta-cyclodextrin.

In another embodiment, the inclusion complex comprises L-Tyrosyl-D-alanyl-glycyl-N-methylphenylalanyl-glycyl-isopropylamide with hydroxypropyl beta-cyclodextrin.

In another embodiment, the inclusion complex comprises L-Tyrosyl-D-alanyl-glycyl-N-methylphenylalanyl-glycyl-isopropylamide with dimethyl beta-cyclodextrin.

In another aspect, the invention provides a pharmaceutical composition comprising a therapeutically effective amount of an inclusion complex according to the invention and a pharmaceutically acceptable carrier.

The pharmaceutical composition according to the invention may be formulated for oral or parenteral administration such as a tablet, capsule, an injectable formulation or for transdermal or rectal administration.

According to another aspect, the invention provides an inclusion complex according to the invention for use as an analgesic.

We have found that the pharmaceutical compositions of the invention exhibit significant analgesic activity with reduced dependence liability, respiratory depression, gastric irritation and sedation, when compared with known narcotic analgesics.

According to a further aspect, the invention provides the use of an inclusion complex according to the invention in the manufacture of a medicament for the treatment of acute inflammation and for alleviating pain.

The L-Tyrosyl-D-alanyl-glycyl-N-methylphenylalanyl-glycylisopropylamide: cyclodextrin (1:2) complex of the invention is used for the oral delivery of the peptide as these complexes provide more protection to the peptide against the gastric environment. The L-Tyrosyl-D-alanyl-glycyl-N-methylphenylalanyl-glycyl-isopropylamide: cyclodextrin (1:1) complex is used for the transdermal delivery of the peptide as these complexes facilitate the transdermal permeation of the peptide.

According to another aspect, the invention provides a process for making an inclusion complex according to the invention, comprising adding L-Tyrosyl-D-alanyl-glycyl-N-methylphenylalanyl-glycyl-isopropylamide to an aqueous solution of the cyclodextrin ; and cooling the mixture to from 0°C to 5°C whereby the complex is precipitated out of the solution.

According to a further aspect of the invention, there is provided a process for making an inclusion complex according to the invention, comprising adding L-Tyrosyl-D-alanyl-glycyl-N-methylphenylalanyl-glycyl-isopropylamide dissolved in methanol to an aqueous solution of the cyclodextrin; and evaporating off the solvents.

In the Examples which follow, reference will be made to the accompanying drawings, by way of illustration only, in which:
FIGURE 1 is a structural formula of L-Tyrosyl-D-alanyl-glycyl-N-methylphenylalanyl-glycyl-isopropylamide;
FIGURE 2 shows a mass spectrum of L-Tyrosyl-D-alanyl-glycyl-N-methylphenylalanyl-glycyl-isopropylamide;
FIGURE 3 shows an NMR spectrum of L-Tyrosyl-D-alanyl-glycyl-N-methylphenylalanyl-glycyl-isopropylamide;
FIGURE 4 shows a differential scanning calorimetry (DSC) thermogram of L-Tyrosyl-D-alanyl-glycyl-N-methylphenylalanyl-glycyl-isopropylamide;
FIGURE 5 shows a DSC thermogram in the curve of the obtained L-Tyrosyl-D-alanyl-glycyl-N-methylphenylalanyl-glycyl-isopropylamide-beta cyclodextrin complex product. There was not detected any endothermic transition for a melting point, characteristic of a physical mixture of L-Tyrosyl-D-alanyl-glycyl-N-methylphenylalanyl-glycyl-isopropylamide/beta-cyclodextrin;
FIGURE 6 shows an NMR spectrum of the complex of L-Tyrosyl-D-alanyl-glycyl-N-methylphenylalanyl-glycyl-isopropylamide with beta-cyclodextrin;
FIGURE 7 shows a mass spectrum of the complex of L-Tyrosyl-D-alanyl-glycyl-N-methylphenylalanyl-glycyl-isopropylamide with beta-cyclodextrin;
FIGURE 8 shows a DSC thermogram in the curve of the obtained L-Tyrosyl-D-alanyl-glycyl-N-methylphenylalanyl-glycyl-isopropylamide-beta cyclodextrin complex product there was not detected any endothermic transition for a melting point, characteristic of a physical mixture of L-Tyrosyl-D-alanyl-glycyl-N-methylphenylalanyl-glycyl-isopropylamide/beta-cyclodextrin;
FIGURE 9 shows an NMR spectrum of the complex of L-Tyrosyl-D-alanyl-glycyl-N-methylphcnytalanyl-glycyl-isopropylamide with beta-cyclodextrin;
FIGURE 10 shows a DSC thermogram of the L-Tyrosyl-D-alanyl-glycyl-N-methylphenylaianyl-glycyl-isopropylamide - betacyclodextrin complex product; and
FIGURE 11 shows NMR spectrum of the complex of L-Tyrosyl-D-alanyl-glycyl-N-methylphenylalanyl-glycyl-isopropylamide with hydroxypropyl-beta-cyclodextrin.

### EXAMPLE 1

### Preparation of L-Tyrosyl-D-alanyl-glycyl-N-methylphenylalanyl-glycyl-isopropylamide

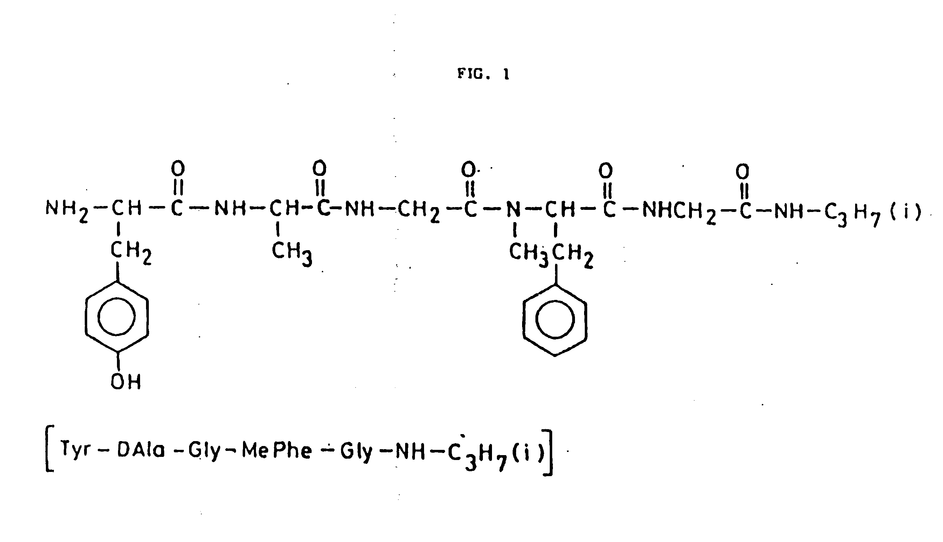

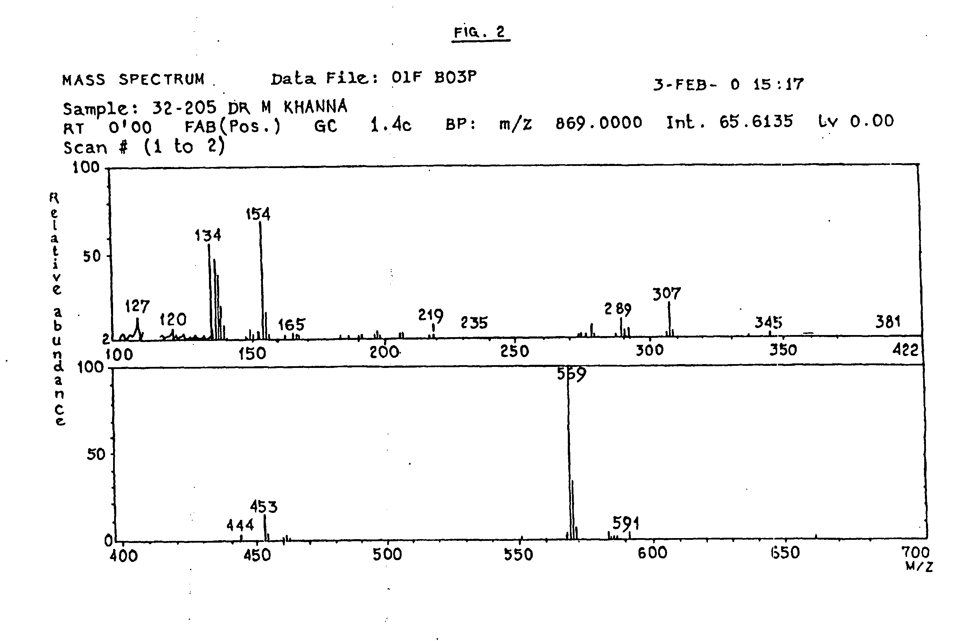

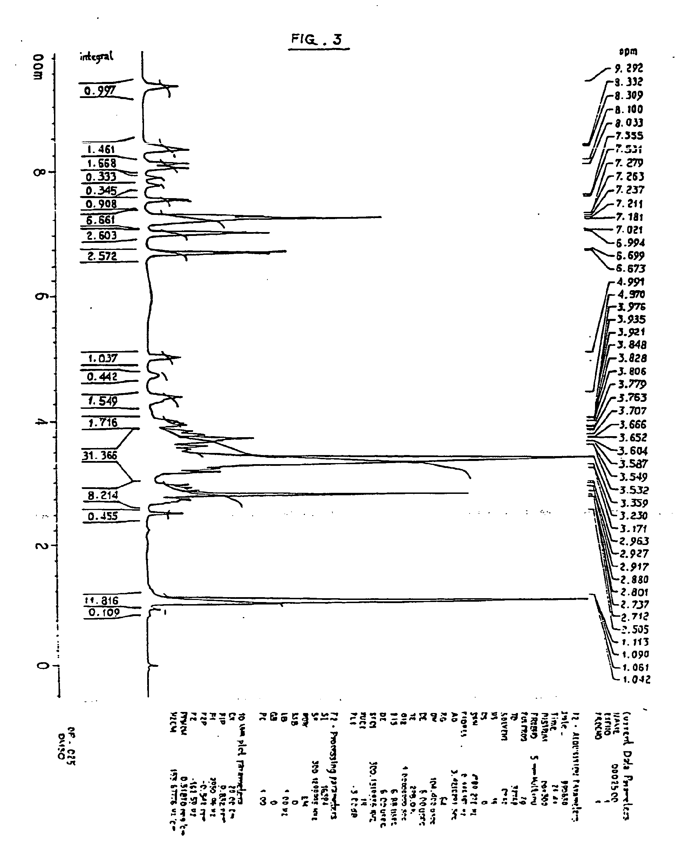

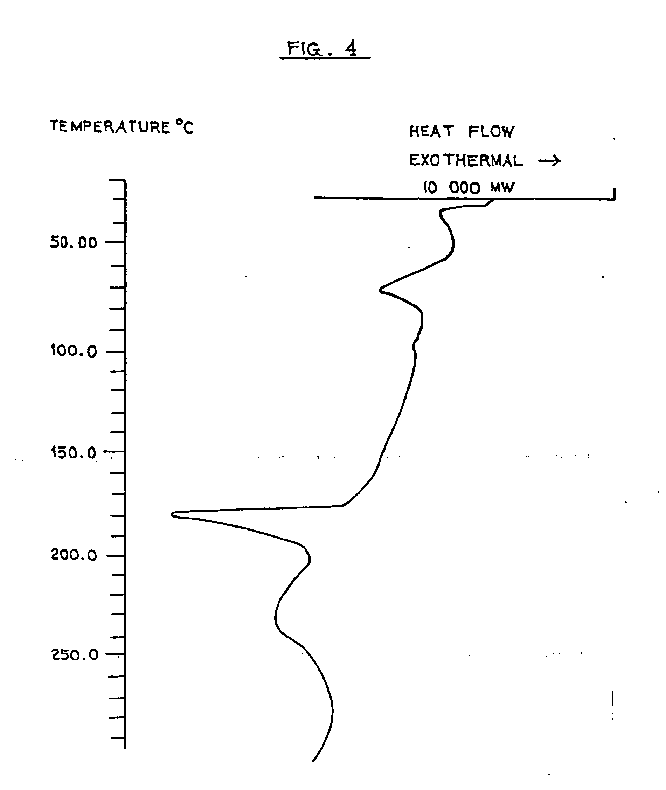

The penta-peptide was synthesised by solution phase method of peptide synthesis employing three plus two fragment condensation method or by step up chain elongation procedure. This compound was also prepared on solid support employing commercially available supports using either BOC or PMOC chemistry The coupling reactions were performed by commonly available reagents adopting reported procedures. The out line of the process for the preparation of the penta-peptide has been disclosed in Indian Patent No. 173568 19.10.1989. Reference is made to Figures 1 to 4 for further information.

### EXAMPLE 2

Preparation of inclusion complex of L-Tyrosyl-D-alanyl-glycyl-N-methylphenylalanylglycyl-isopropylamide with beta-cyclodextrin

### a) Procedure in aqueous medium

Beta-cyclodextrin (1.135 g, 1.0 m mole) in water (25 ml) was heated to boiling temperature. L-Tyrosyl-D-alanyl-glycyl-N-methylphenyl-alanyl-glycyl-isopropylamide (0 568 g, 1.0 m mole) was added into this solution, vigorously stirred for 15 minutes and the mixture was cooled during stirring to 2 temperature between 0 degree and 5 degree centigrade. The obtained complex was dried in vacuum at the temperature of about 40 degree C. Inclusion complex (1.62 g, 95.1%) of L-Tyrosyl-D-alanyl-glycyl-N-methylphenylalanyl-glycyl-isopropylamide with beta-cyclodextrin was obtained in the form of a white powder in the molar ratio of 1:1.

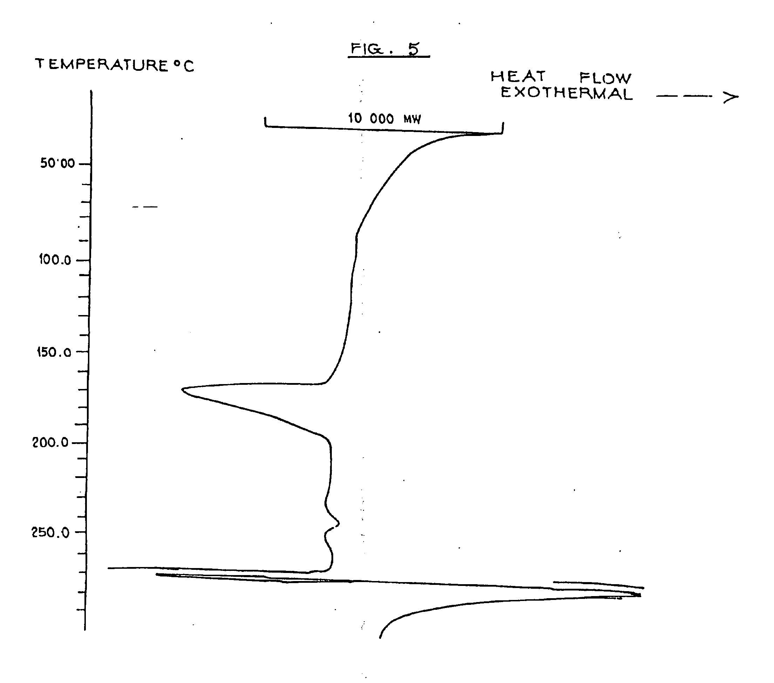

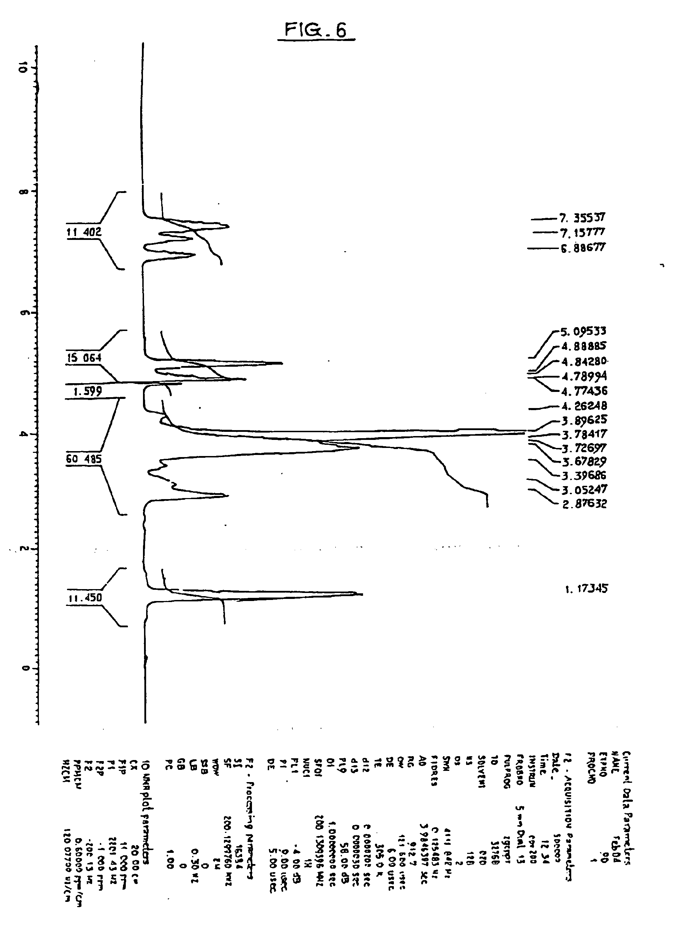

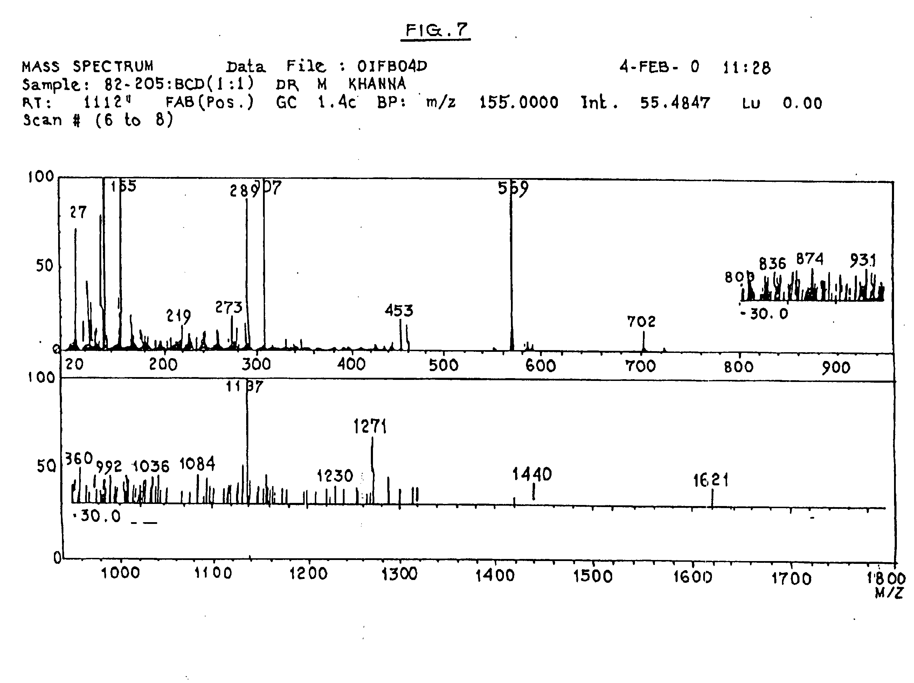

Data on reaction yields. L-Tyrosyl-D-alanyl-glycyl-N-methylphenylalanyl-glycyl-isopropylamide content in the complex (determined theoretically and experimentally-spectrophotometric determination at the wavelength of 275 nm) are summarised in Table 1.Reference is made to Figures 5,6 and 7 for further information.

### b) Procedure in a solvent mixture (methanol/water in the ratio 5-20)

Beta-cyclodextrin (1 135 g; 1.0 m mole) was dissolved in water (25 ml) at a temperature of about 70 degree C. During stirring a solution of L-Tyrosyl-D-alanyl-glycyl-N-methylphenylalanyl-glycyl-isopropylamide (0.568 g; 1.0 m mole) in methanol (5 ml) was added. It was stirred for another 5 minutes, the solvents were evaporated and the obtained complex was dried in vacuum at a temperature about 40 degree C. Inclusion complex (1.59 g, 93.4%) of L-Tyrosy-D-alanyl-glycyl-N-methylphenylalanyl-glycyl-isoprophlamide with beta-cyclodextein was obtained in the form of a white powder in the molar ratio of 1:1. Differential scanning calorimetry, mass and NMR spectrum showed the same results as in the process for preparing the inclusion complex in an aqueous medium.

### EXAMPLE 3

Preparation of inclusion complex of L-Tyrosyl-D-alanyl-glycyl-N-methylphenylalanyl-glycyl-isopropylamide with beta-cyclodextrin

### a) Procedure in aqueous medium

Beta-cyclodextrin (1 135 g; 1.0 m mole) in water (25 ml) was heated to boiling temperature, into the boiling solution of L-Tyrosyl-D-alanyl-glycyl-N-methylphenyl-alanyl-glycyl-isopropylamide (0.284 g; 0.5 m mole) was added, vigorously stirred for 15 minutes and the mixture was cooled during stirring to a temperature between 0 degree and 5 degree C. The obtained complex was dried in vacuum at the temperature of about 40 degree C. Inclusion complex (1.33 g; 93.7%) of L-Tyrosyl-D-alanyl-glycyl-N-methylphenyl-alanyl-glycyl-isopropylamide with beta.-cyclodextrin was obtained in the form of a white powder in the molar ratio of 1:2.

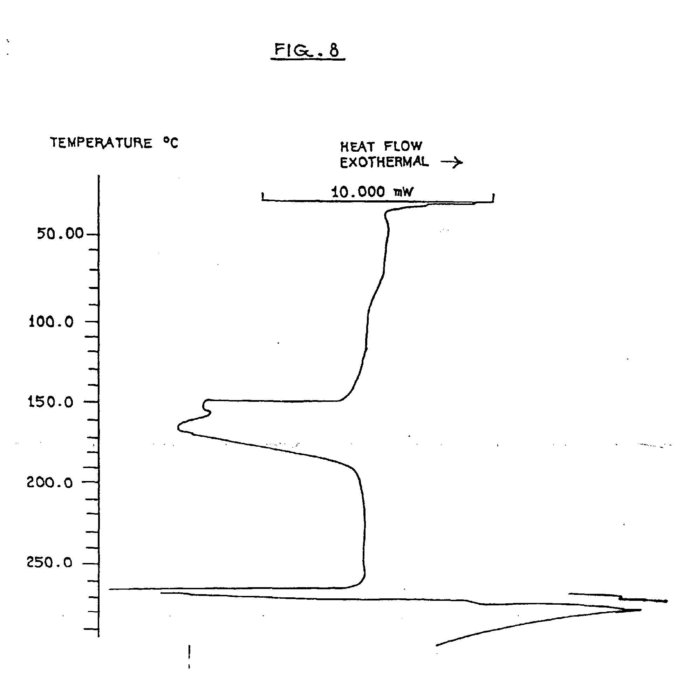

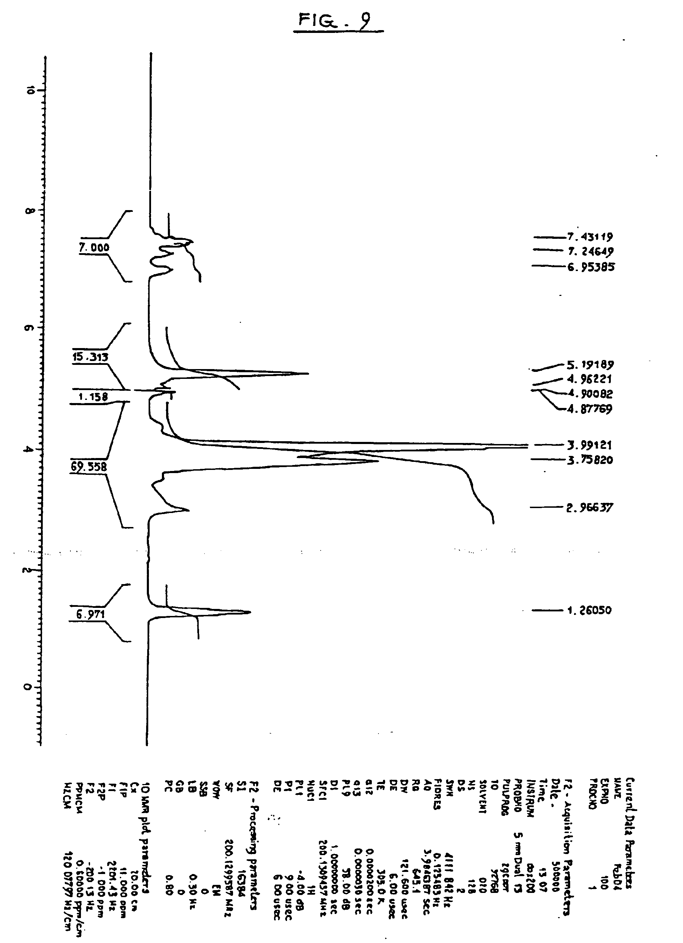

Data on reaction yields, L-Tyrosyl-D-alanyl-glycyl-N-methylphenylalanyl-glycyl-isopropylamide content in the complex (determined theoretically and experimentally-spectrophotometric determination at the wavelength of 275 nm) are summarised in table 1. Reference is also made to Figures 8 and 9 for further information.

### b) Procedure in a solvent mixture (methanol/water in the ratio 5:20)

Beta-cyclodextrin (1 135 g; 1.0 m mole) was dissolved in water (25 ml) at a temperature of about 70 degree C. L-Tyrosyl-D-alanyl-glycyl-N-methylphenylalanyl-glycyl-isopropylamide (0.284 g; 0.5 m mole) in methanol (5 ml) was added and stirred for another 5 minutes. The solvents were evaporated and the complex obtained was dried in vacuum at a temperature about 40 degree C. Inclusion complex (1.36 g; 95.8%) of L-Tyrosyl-D-alanyl-glycyl-N-methylphenylalanyl-glycyl-isopropylamide with beta.-cyclodextrin was obtained in the form of a white powder in the molar ratio of 1:2.
Differential scanning calorimetry and NMR spectrum showed the same results as in the process for preparing the inclusion complex in an aqueous medium.

### EXAMPLE 4

Preparation of inclusion complex of L-Tyrosyl-D-alanyl-glycyl-N-methylphenylalanyl-glycyl-isopropylamide with hydroxypropyl-beta-cyclodextrin

### a) Procedure in methanolic medium

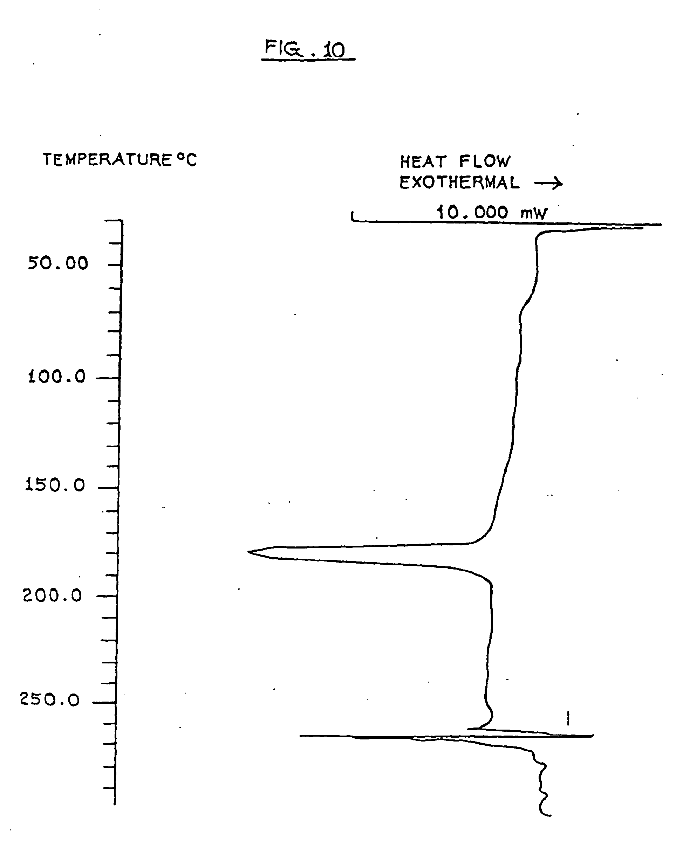

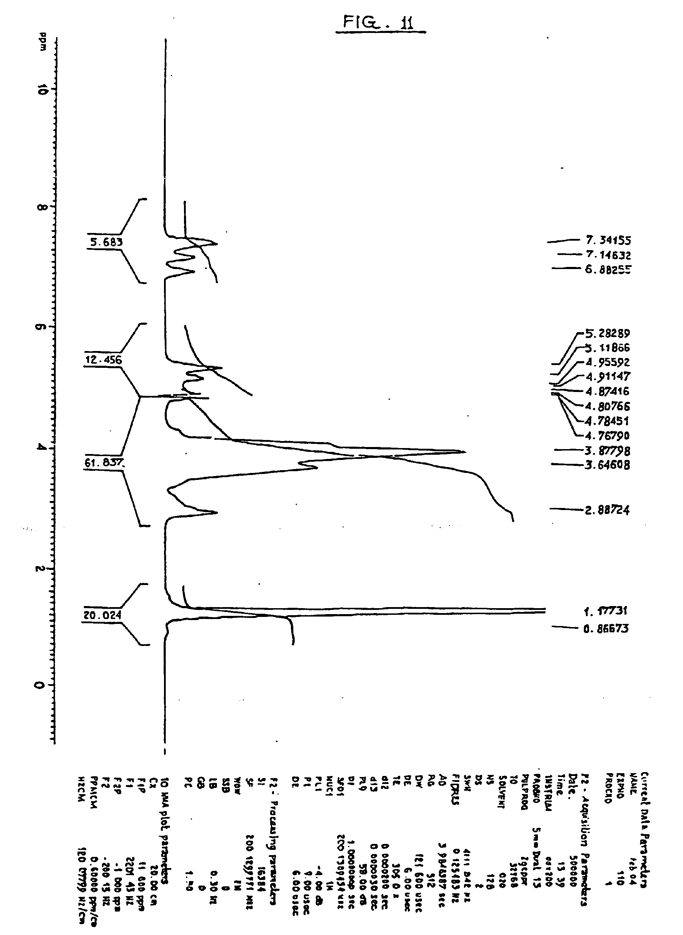

L-Tyrosyl-D-alanyl-glycyl-N-methylphenylalanyl-glyoyl-isopropylamide (0 568 g, 1.0 m mole) was added to a solution of hydroxypropyl-beta-cyclodextrin (1.38 g; 1.0 m mole) in methanol (25 ml) and the obtained solution was stirred for another 5 minutes at room temperature Methanol was then evaporated and the obtained complex was dried in vacuum at the temperature of 40 degree C. Inclusion complex (1.82g; 93.4%) of L-Tyrosyl-D-alanyl-glycyl-N-methylphenylalanyl-glycyl-isopropylamide with hydroxypropyl-beta-cyclodextrin was obtained in the form of a white powder in the molar ratio of 1:1.
Data on reaction yields, L-Tyrosyl-D-alanyl-glycyl-N-methylphenylalanyl-glycyl-isopropylamide content in the complex (determined theoretically and experimentally-spectrophotometric determination at the wavelength of 275 nm) of the complex formed are summarised in Table 1. Reference is made to Figures 10 and 11 for further information.

### b) Procedure in aqueous medium

Hydroxypropyl-beta-cyclodextrin (1.38 g; 1.0 m mole) was dissolved in water (40 ml) and the obtained solution was heated to the temperature of 70.degree C and L-Tyrosyl-D-alanyl-glycyl-N-methylphenylalanyl-glycyl-isopropylamide (0.568 g; 1.0 m mole) was added. It.was vigorously stirred for another 15 minutes and then the solution was filtered The filtrate was frozen in liquid nitrogen and lyophilised. Inclusion complex (1.81g; 92.9 %) of L-Tyrosyl-D-alanyl-glycyl-N-methylphenylalanyl-glycyl-isopropylamide with hydroxypropyl-beta-cyclodextrin was obtained in the form of a white powder in the molar ratio of 1:1.
Differential scanning calorimetry and NMR spectrum showed the same results as in the process for preparing inclusion complex in the methanolic medium.

### EXAMPLE 5

Preparation of inclusion complex of L-Tyrosyl-D-alanyl-glycyl-N-methylphenylalanyl-glycyl-isopropylamide with hydroxyethyl-beta-cyclodextrin

### a) Procedure in methanolic medium

L-Tyrosyl-D-alanyl-glycyl-N-methylphenylalanyl-glycyl-isopropylamide (0.568 g.1.0. mmole) was added to a solution of hydroxyethyl-beta -cyclodextrin (1.44 g; 1.0 mmole) in methanol (10 ml) and the obtained solution was stirred for another 5 minutes at room temperature. Methanol was then evaporated and the obtained complex was dried in vacuo at the temperature of 40 degree C. Inclusion complex (1.87 g; 93.1 %) of L-Tyrosyl-D-alanyl-glycyl-N-methylphenylalanyl-glycyl-isopropylamide with hydroxyethyl-.beta.-cyclodextrin was obtained in the form of a white powder in the molar ratio of 1:1.
Differential scanning calorimetry and NMR spectrum showed the formation of inclusion complex.

### b) Procedure in aqueous medium

Hydroxyethyl-beta-cyclodextrin (1.44 g; 1.0 mmole) was dissolved in water (40 ml). The obtained solution was heated to the temperature of 70.degree. C. and L-Tyrosyl-D-alanyl-glycyl-N-methylphenylalanyl-glycyl-isopropylamide (0.568 g; 1.0 mmole) was added. It was vigorously stirred for another 15 minutes and then the solution was filtered. The filtrate was frozen in liquid nitrogen and lyophilized Inclusion complex (1.89 g; 94.1%)of Tyrosyl-D-alanyl-glycyl-N-methylphenylalanyl-glycyl-isopropylamide with hydroxyethyl-beta-cyclodextrin was obtained in the form of a white powder in the molar ratio of 1:1.
Differential scanning calorimetry and NMR spectrum showed the formation of inclusion complex

**TABLE 1**

| Reaction yield, L-Tyrosyl-D-alanyl-glycyl-N-methylphenylalanyl-glycyl-isopropylamide content in the complex for inclusion complex of L-Tyrosyl-D-alanyl-glycyl-N-methylphenylalanyl-glycyl-isopropylamide with different derivatives of beta-cyclodextrin. | | |
|---|---|---|
| Inclusion complex | Reaction yield (%) | % Content of L-Tyrosyl-D-alanyl-glycyl-N-methylphenylalanyl-glycyl-isopropylamide in the complex |
| Beta-cyclodextrin (1:1) | 95.1 | 97.2 |
| Beta-cyclodextrin (1:2) | 93.7 | 92.88 |
| hydroxypropyl-beta-cyclodextrin (1:1) | 93.4 | 98.7 |
| hydroxyethyl-beta-cyclodextrin (1:1) | 93.1 | 97.5 |

### EXAMPLE 6

30 days toxicity study of L-Tyrosyl-D-alanyl-glycyl-N-methylphenylanyl-glycyl-isopropylamide in rat and rhesus monkey by subcutaneous route.

Compound L-Tyrosyl-D-alanyl-glycyl-N-methylphenylalanyl-glycyl-isopropylamide dissolved in saline was given daily by subcutaneous route to groups of rats (n=10 males and 10 females in each group) and rhesus monkeys (n=2 males and 2 females in each group) once daily for 30 consecutive days. The dose levels used in rats were 0 0, 7.5, 15.0 and 30.0 mg/kg and in monkeys 0.0, 3.75, 7.5 and 15 mg/kg. Dose levels were selected on the basis of rat ED of 3.0 mg/kg and extrapolation thereof in the monkey.

A careful general inspection of the animals was done at least once each day Cage-side observations included changes in skin and fur, eyes and mucous membranes, general activity and behavior and also general signs of abnormal functioning of respiratory, circulatory, autonomic and central nervous system and somatomotor activity.

Body weights (weekly in rats, initial and day 30 in monkeys), daily food (rat and monkeys) and water (rat only) consumption and parameters of hematology, urinalysis and blood biochemistry of the animals was recorded All the animals were sacrificed at the end of 30 days Necropsy was performed and histophathological examination of all the important organs and tissues were done.

Urinalysis included the recording of colour, specific gravity reaction (pH) and testing for sugar, acetone, urobillinogen, bilirubin, and albumin and occult blood Microscopic examination of urinary sediment was done for the presence of epithelial cells, WBCs, RBCs, casts, crystals and other abnormal constituents.

The hematological examinations included hemoglobin, T-RBC count mean corpuscular hemoglobin concentration, mean corpuscular volume, total leukocyte count, packed cells volume mean corpuscular volume, platelet counts, differential leukocyte counts, erythrocyte sedimentation rate (monkeys only) and prothrombin time test (monkeys only).

Biochemical estimations in blood done at day 30 in rat, and day 0 and 30 in monkeys) included glucose, creatinine, urea nitrogen, sodium, potassium SGPT (ALT), alkaline phosphatase (ALP), bilirubin, cholesterol, total serum proteins, albumin and globulin.

During necropsy examination of the external surface of the body, all orifices, and the cranial thoracic and abdominal cavities and their contents was performed. A thorough naked eye examination of size, shape, surface, colour, contours etc of all the important organs and tissues was done. Liver, kidneys, adrenals, brain heart lungs, spleen and gonads were weighed, and their rdative weights were also calculated.

All the above mentioned organs, and representative pieces from skin trachea, thyroid, different parts of the GIT pancreas, urinary bladder, mammary glands and whole eyes were preserved in 10% buffered formalin till they were processed for paraffin embedding and sectioning.

Representative places from all the preserved organs and tissues were processed for paraffin embedding. Four to six micrometer thick sections are cut and stained by haematoxylin and eosin according to standard methods for microscopic examination.

The animals continued to remain active and healthy throughout the period of experimentation. Animals of both drug treated and control groups showed uniform (rats) or irregular (monkeys) trends of gain in body weight. The laboratory investigations showed some minor incidental variations but there was no indication of drug induced damages in the various urinalyses hematological and bloods biochemical values. Also, necropsy (including absolute and relative organ weights of important organs and histopathological examinations did not reveal any sign of target organ toxicity.
Compound L-Tyrosyl-D-alanyl-glycyl-N-methyphenylalanyl-glycyl-ispropylamide was thus found safe in rats and rhesus monkeys in the 30-day toxicity study by subcutaneous route at the dose levels mentioned above.

### EXAMPLE 7

### Alleviation of pain:

Analgesia was measured by tail flick latencies (t.f.l.) assay in rats. (D'Amour, F. E and Smith., D. L., *J*. Pharmacol. Exp. Ther. 72,74-79, 1941] The test required determination of tail flick latencies to heat stimulus. The basal tail flick latencies were determined twice at 10 min interval and averaged to obtain single pre drug latency. An increase in the latency by 100% or more was indicative of analgesic state of animal. A cut-off time of 10 seconds was used to avoid damage to the tail skin.

The test compounds were administered (subcutaneously or per oral) in graded dose in-groups of 8-10 rats each. Then tail flick latenoy was determined every 10 minutes till it was near the pre drug level. Percent of animals exhibiting analgesia was determined at each dose level of various compounds and the ED₅₀ along with 95% fiducial limits was calculated [Table 2] by prohibit analysis (Finney, 1971), The duration of analgesic effect was determined at the peat effect. The analgesia lasted for 5-6 hours after oral administration.

**TABLE 2**

| Compound/Formulation | ED₅₀(mg/Kg) | |
|---|---|---|
| | S.C. route | P. O. route |
| L-Tyrosyl-D-alanyl-glycyl-N-methylphenylalanyl-glycyl-isopropylamide | 2.58 | 22.30 |
| L-Tyrosyl-D-alanyl-glycyl-N-methylphenylalanyl- glycyl-isopropylamide: beta CD complex (1:2) | 4.45 (equivalent to 0.89 mg of compound) | 54.23 (equivalent to 10.84 mg of compound) |

It is evident from above table that the ED₅₀ of beta CD complex is about one third than the compound L-Tyrosyl-D-alanyl-glycyl-N-methylphenylalanyl-glycyl-isopropylamide by subcutaneous route and was one half by oral route. The LD₅₀ of the compound L-Tyrosyl-D-alanyl-glycyl-N-methylphenylalanyl-glycyl-isopropylamide is 1000 mg/Kg *i.p* and that of the complex is> 10,000 mg/kg P. O. in mice.

### EXAMPLE 8

### Anti-inflammatory action

Anti-inflammatory action was measured *in vivo* by the inhibition of oedema caused by carrageenin Rats were given 250/500 mg/kg of the test substance 1 hour before the injection of 0.1 ml 1% carrageenin suspension. The inhibition of the formed oedema was measured 3 hours after injecting carrageenin. [Table 3]

**TABLE 3**

| Measurement of anti-inflammatory action *in vivo* at the dosages of 500/250 mg/kg of the active substance applied per orally substance | | |
|---|---|---|
| | Dose (p.o.) (mg/kg) | Anti-inflammatory action (210 min) |
| L-Tyrosyl-D-alanyl-glycyl-N-methylphenylalanyl-glycyl-isopropyl amide:beta CD complex (1:2) | 250 | 4.0 |
| L-Tyrosyl-D-alanyl-glycyl-N-methylphenylalanyl-glycyl-isopropyl amide:beta CD complex (1:2) | 500 | 18.4. |
| L-Tyrosyl-D-alanyl-glycyl-N-methylphenylalanyl-glycyl-isopropyl amide | 50 | 19.6 |

The measurement *in vivo* of the anti-inflammatory action showed that the inclusion complex of L-Tyrosyl-D-alanyl-glycyl-N-methylphenylalanyl-glycyl-isopropylamide with beta cyclodextrin exhibited anti-inflammatory action.

### EXAMPLE 9

### Effect on gastric mucous membrane

Effect of L-Tyrosyl-D-alanyl-glycyl-N-methylphenylalanyl-glycyl-isopropylamide and inclusion complex of L-Tyrosyl-D-alanyl-glycyl-N-methylphenylalanyl-glycyl-isopropylamide with beta cyclodextrin on gastric mucous membrane was measured in rats. Rats were fasted overnight and were given 100 mg/kg of the active substance orally. After 4 hours its effect on irritation of gastric mucous membrane and gastric ulceration, gastric juice volume and extent of free and total acid was determined. [Table 4].

**TABLE 4**

| Measurement of the effect of L-Tyrosyl-D-alanyl-glycyl-N-methylphenylalanyl-glycyl-isopropylamide beta-cyclodextrin (1:1) on gastric mucous membrane | | | | |
|---|---|---|---|---|
| Dose (mg/kg) | Ulceration | Gastric Juice volume | Free acid | Total acid |
| 100 | - | - 23 | + 9.0 | + 10.0 |

The above data show that L-Tyrosyl-D-alanyl-glycyl-N-methylphenylalanyl-glycyl-isopropylamide bound into an inclusion complex with beta.-cyclodextrin did not exhibit an irritating effect on gastric mucous membrane of the animals.

### EXAMPLE 10

Preparation of tablets with 125 mg of active substance [inclusion complex (1:2)] of L-Tyrosyl-D-alanyl-glycyl-N-methylphenylalanyl-glycyl-isopropylamide with beta-cyclodextrin
Tablets of the following composition were prepared

**TABLE 5**

| Active constituent | weight |
|---|---|
| L-Tyrosyl-D-alanyl-glycyl-N-methylphenylalanyl-glycyl-isopropylamide :beta cyclodextrin complex (1:2) | 125 mg |
| lactose | 70 mg |
| methyl cellulose | 5 mg |
| magnesium stearate | 2mg |

Preparation of tablets: The active substance was homogeneously stirred with additives. The mixture was sicved through a sieve and pressed into tablets on a rotating tableting machine and the resulting tablet properly packed in a polythene lined blister type packing to avoid coming into contact with moisture before use.

### EXAMPLE 11

Preparation of transdermal tapes of active substance (inclusion complex (1:1)) of L-Tyrosyl-D-alanyl-glycyl-N-methylphenylalanyl-glycyl-isopropylamide with beta-cyclodextrin

Polyvinyl alcohol (4 g) and polyvinylpyrrolidone (2 g) were taken in a beaker and stirred at 75° C, 120 ml ethyl alcohol (50% v/v) was slowly added into this to get a fine latex. L-Tyrosyl-D-alanyl-glycyl-N-methylphenylalanyl-glycyl-isopropylamide.beta- cyclodextrin complex (1:1) (1.8) g was taken & suspended in propylene glycol (2 ml) PEG 400 (0.5 ml), poly propylene glycol (2 ml) and tritan X-100 (1.5 ml) by using sonicator Polymer latex prepared in earlier step was transferred to it by continuous sterring and further stirred for 15 minutes The hydrogel so formed was uniformly poured into a petri dish and allowed to dry The next day a yellow coloured patch was obtained This was covered with the plastic film and cut into the desired size. The patches so obtained were properly packed in a polythene lined blister type packing to avoid coming into contact with moisture before use.

## Claims

1. An inclusion complex comprising a complex of L-Tyrosyl-D-alanyl-glycyl-N-methylphenylalanyl-glycyl-isopropylamide with a cyclodextrin, wherein the molar ratio between L-Tyrosyl-D-alanyl-glycyl-N-methylphenylalanyl-glycyl-isopropylamide and the cyclodextrin is from 1:5 to 2:1.

2. An inclusion according to claim 1, wherein the cyclodextrin is beta cyclodextrin, hydroxypropyl-beta cyclodextrin, dimethyl-beta cyclodextrin or hydroxyethyl-beta-cyclodextrin.

3. A pharmaceutical composition comprising a therapeutically effective amount of inclusion complex according to any preceding claim and a pharmaceutically acceptable carrier.

4. A pharmaceutical composition according to claim 3, formulated for oral or parenteral administration.

5. A pharmaceutical composition according to claim 4, wherein the oral form is a tablet or capsule.

6. A pharmaceutical composition according to claim 4, wherein the parenteral form is an injectable composition, or is for transdermal or rectal administration.

7. An inclusion complex according to claim 1 or 2, for use as an analgesic.

8. The use of an inclusion complex according to claim 1 or 2 in the manufacture of a medicament for the treatment of acute inflammation and for alleviating pain.

9. The use according to claim 8, wherein the inclusion complex comprises a complex of L-Tyrosyl-D-alanyl-N-methylphenylalanyl-glycyl-isopropylamide with beta-cyclodextrin and the medicament is for oral or topical administration.

10. A process for making an inclusion complex according to claim 1 or 2 comprising adding L-Tyrosyl-D-alanyl-glycyl-N-methylphenylalanyl-glycyl-isopropylamide to an aqueous solution of the cyclodextrin ; and cooling the mixture to from 0°C to 5°C whereby the complex is precipitated out of the solution.

11. A process for making an inclusion complex according to claim 1 or 2, comprising adding L-Tyrosyl-D-alanyl-glycyl-N-methylphenylalanyl-glycyl-isopropylamide dissolved in methanol to an aqueous solution of the cyclodextrin ; and evaporating off the solvents.

12. An inclusion complex according to claim 1, comprising L-Tyrosyl-D-alanyl-glycyl-N-methylphenylalanyl-glycyl-isopropylamide and beta-cyclodextrin having a differential scanning calorimetry thermogram according to Figure 5, an NMR spectrum according to Figure 6 and a mass spectrum according to Figure 7.

13. An inclusion complex according to claim 1, comprising L-Tyrosyl-D-alanyl-glycyl-N-methylphenylalanyl-glycyl-isopropylamide and beta-cyclodextrin having a differential scanning calorimetry thermogram according to Figure 8 and an NMR spectrum according to Figure 9.

14. An inclusion complex according to claim 1, comprising L-Tyrosyl-D-alanyl-glycyl-N-methylphenylalanyl-glycyl-isopropylamide and hydroxypropyl-beta-cyclodextrin having a differential scanning calorimetry thermogram according to Figure 10 and an NMR spectrum according to Figure 11.

## Patentansprüche

1. Einschlusskomplex, umfassend einen Komplex aus L-Tyrosyl-D-alanyl-glycyl-N-methylphenylalanyl-glycylisopropylamid mit einem Cyclodextrin, worin das Molverhältnis zwischen L-Tyrosyl-D-alanyl-glycyl-N-methylphenylalanyl-glycyl-isopropylamid und dem Cyclodextrin von 1:5 bis 2:1 beträgt.

2. Einschluss nach Anspruch 1, worin das Cyclodextrin β-Cyclodextrin, Hydroxypropyl-β-cyclodextrin, Dimethyl-β-cyclodextrin oder Hydroxyethyl-β-cyclodextrin darstellt.

3. Pharmazeutische Zusammensetzung, umfassend eine therapeutisch wirksame Menge des Einschlusskomplexes nach einem der vorangehenden Ansprüche und einen pharmazeutisch verträglichen Träger.

4. Pharmazeutische Zusammensetzung nach Anspruch 3, die zur oralen oder parenteralen Verabreichung formuliert ist.

5. Pharmazeutische Zusammensetzung nach Anspruch 4, worin die orale Form eine Tablette oder Kapsel darstellt.

6. Pharmazeutische Zusammensetzung nach Anspruch 4, worin die parenterale Form eine injizierbare Zusammensetzung darstellt oder zur transdermalen oder rektalen Verabreichung vorgesehen ist.

7. Einschlusskomplex nach Anspruch 1 oder 2 zum Gebrauch als ein Analgetikum.

8. Verwendung eines Einschlusskomplexes nach Anspruch 1 oder 2 bei der Herstellung eines Arzneimittels für die Behandlung einer akuten Entzündung und zur Linderung von Schmerzen.

9. Verwendung nach Anspruch 8, worin der Einschlusskomplex einen Komplex aus L-Tyrosyl-D-alanyl-N-methylphenylalanyl-glycyl-isopropylamid mit β-Cyclodextrin umfasst und das Arzneimittel zur oralen oder topischen Verabreichung vorgesehen ist.

10. Verfahren zur Herstellung eines Einschlusskomplexes nach Anspruch 1 oder 2, umfassend das Zufügen von L-Tyrosyl-D-alanyl-glycyl-N-methylphenylalanyl-glycylisopropylamid zu einer wässrigen Lösung aus dem Cyclodextrin; und Abkühlen des Gemischs von 0 °C bis 5 °C, wobei der Komplex aus der Lösung ausgefällt wird.

11. Verfahren zur Herstellung eines Einschlusskomplexes nach Anspruch 1 oder 2, umfassend das Zufügen von L-Tyrosyl-D-alanyl-glycyl-N-methylphenylalanyl-glycylisopropylamid, das in Methanol zu einer wässrigen Lösung aus dem Cyclodextrin aufgelöst ist; und Abdampfen der Lösungsmittel.

12. Einschlusskomplex nach Anspruch 1, umfassend L-Tyrosyl-D-analyl-glycyl-N-methylphenylalanyl-glycylisopropylamid und β-Cyclodextrin mit einem Differenzial-Scanning-Kalorimetrie-Thermogramm nach Figur 5, einem NMR-Spektrum nach Figur 6 und einem Massenspektrum nach Figur 7.

13. Einschlusskomplex nach Anspruch 1, umfassend L-Tyrosyl-D-alanyl-glycyl-N-methylphenylalanyl-glycylisopropylamid und β-Cyclodextrin mit einem Differenzial-Scanning-Kalorimetrie-Thermogramm nach Figur 8 und einem NMR-Spektrum nach Figur 9.

14. Einschlusskomplex nach Anspruch 1, umfassend L-Tyrosyl-D-alanyl-glycyl-N-methylphenylalanyl-glycylisopropylamid und Hydroxypropyl-β-cyclodextrin mit einem Differenzial-Scanning-Kalorimetrie-Thermogramm nach Figur 10 und einem NMR-Spektrum nach Figur 11.

## Revendications

1. Complexe d'inclusion comprenant un complexe de L-tyrosyl-D-alanyl-glycyl-N-méthylphénylalanyl-glycyl-isopropylamide avec une cyclodextrine, dans lequel le rapport molaire entre le L-tyrosyl-D-alanyl-glycyl-N-méthylphénylalanyl-glycyl-isopropylamide et la cyclodextrine est de 1:5 à 2:1.

2. Inclusion selon la revendication 1, dans laquelle la cyclodextrine est la bêta-cyclodextrine, l'hydroxypropyl-bêta-cyclodextrine, la diméthyl-bêta-cyclodextrine ou l'hydroxyéthyl-bêta-cyclodextrine.

3. Composition pharmaceutique comprenant une quantité d'efficacité thérapeutique de complexe d'inclusion selon l'une quelconque des revendications précédentes et un excipient acceptable en utilisation pharmaceutique.

4. Composition pharmaceutique selon la revendication 3, préparée pour l'administration orale ou parentérale.

5. Composition pharmaceutique selon la revendication 4, dans laquelle la forme orale est un comprimé ou une capsule.

6. Composition pharmaceutique selon la revendication 4, dans laquelle la forme parentérale est une composition injectable ou est prévue pour l'administration transdermique ou rectale.

7. Complexe d'inclusion selon la revendication 1 ou 2, pour utilisation comme analgésique.

8. Utilisation d'un complexe d'inclusion selon la revendication 1 ou 2 dans la fabrication d'un médicament pour le traitement de l'inflammation aiguë et pour le soulagement de la douleur.

9. Utilisation selon la revendication 8, dans laquelle le complexe d'inclusion est un complexe de L-tyrosyl-D-alanyl-N-méthylphénylalanyl-glycyl-isopropylamide avec de la bêta-cyclodextrine et dans laquelle le médicament est prévu pour l'administration orale ou topique.

10. Procédé pour fabriquer un complexe d'inclusion selon la revendication 1 ou 2 comprenant l'adjonction de L-tyrosyl-D-alanyl-glycyl-N-méthylphénylalanyl-glycyl-isopropylamide à une solution aqueuse de la cyclodextrine, et le refroidissement du mélange à une température de 0°C à 5°C, grâce à quoi le complexe est précipité hors de la solution.

11. Procédé pour fabriquer un complexe d'inclusion selon la revendication 1 ou 2, comprenant l'adjonction de L-tyrosyl-D-alanyl-glycyl-N-méthylphénylalanyl-glycyl-isopropylamide dissous dans du méthanol à une solution aqueuse de la cyclodextrine, et l'évaporation des solvants.

12. Complexe d'inclusion selon la revendication 1, comprenant du L-tyrosyl-D-alanyl-glycyl-N-méthylphénylalanyl-glycyl-isopropylamide et de la bêta-cyclodextrine ayant un thermographe d'analyse calorimétrique différentielle selon la Figure 5, un spectre de résonance magnétique nucléaire selon la Figure 6, et un spectre de masse selon la Figure 7.

13. Complexe d'inclusion selon la revendication 1, comprenant du L-tyrosyl-D-alanyl-glycyl-N-méthylphénylalanyl-glycyl-isopropylamide et de la bêta-cyclodextrine ayant un thermographe d'analyse calorimétrique différentielle selon la Figure 8 et un spectre de résonance magnétique nucléaire selon la Figure 9.

14. Complexe d'inclusion selon la revendication 1, comprenant du L-tyrosyl-D-alanyl-glycyl-N-méthylphénylalanyl-glycyl-isopropylamide et de l'hydroxypropyl-bêta-cyclodextrine ayant un thermographe d'analyse calorimétrique différentielle selon la Figure 10 et un spectre de résonance magnétique nucléaire selon la Figure 11.
